# EUROPEAN PATENT APPLICATION

(11) **EP 3 167 794 A1**
(43) Date of publication of application: **17.05.2017**
(21) Application number: 15193988.1
(22) Date of filing: 10.11.2015
(51) Int. Cl.: A61B 5/00, A61B 5/11, A61B 5/024

(54) **PHYSIOLOGICAL DETECTION STRUCTURE**

(71) Applicant: King's Metal Fiber Technologies Co., Ltd., 42060 Taichung City (TW)
(72) Inventor: KANG, Yu Hsun, 10451 Taipei City (TW); CHANG, Li Chuan, 10451 Taipei City (TW); WANG, Hao Chen, 10451 Taipei City (TW); LIAO, Shu Fen, 10451 Taipei City (TW); CHEN, Reng Sho, 10451 Taipei City (TW)
(74) Representative: Cabinet Chaillot

(57) **Abstract**

A physiological detection structure includes a main body, a slidable member, and a detection module. The slidable member is combined to a surface of the main body. The main body includes at least one opening formed therein. The slidable member has an extra preserved length. The detection module is combined to the slidable member and exposed outside the opening of the main body. As such, the detection module is allowed to displace by means of the extra preserved length of the slidable member to provide the detection module with an effect of changing the site where detection is to be made for detecting physiological signals of different sites of the human body. Further, with such an operatively-disconnected arrangement, the stability of the detection module is ensured and discomfort of human body caused by the detection module being long retained on and depressing a fixed location of the skin is eliminated.

## Description

### BACKGROUND OF THE INVENTION

### 1. Field of the Invention

The present invention relates to a physiological detection structure, and more particularly to one that comprises a combination of a main body, a slidable member, and a detection module, in which the detection module is provided with an effect of changing a detection site so as to eliminate the phenomenon of inflaming caused by the skin being long depressed at a fixed location and it being applicable to a physiological detective garment, an inclination detective garment, or the likes.

### 2. Description of Related Art

The progress of science and technology makes it possible to develop a combination of detection modules with clothing in order to facilitate inspect and record the physiological conditions of a human body and allows for applications to self-management of home healthcare or preventive medical treatments.

Heretofore, the combination of a detection module with clothing is generally fixed. In other words, the detection module is retained, through sewing, at a location, such as chest, where detection is to be made in order to allow the detection module to engage a skin surface of a human body for detection of physiological signals of the human body.

Further, in detecting a physiological signal, in order not to have the detection interrupted where the signal detected is not continuous, the detection module must be kept constantly in contact with a surface of a skin.

The conventionally adopted way of having the detection module combined with clothing in a fixed manner would keep the detection module tightly engaging the skin surface due to the condition of the clothing being worn long. This might lead to inflaming of the skin surface and makes the user uncomfortable.

Thus, in view of the above problems, the present invention aims to provide a physiological detection structure that provides the detection module with an effect of easily changing the detection site and allows for easy operation and installation by a user.

### SUMMARY OF THE INVENTION

The primary object of the present invention is to provide a physiological detection structure, which comprises a combination of a main body, a slidable member, and a detection module. The main body is provided with at least one opening formed therein. The slidable member is combined to a surface of the main body and the slidable member is provided with an extra preserved length. The detection module is combined to the slidable member and the detection module is exposed outside the opening of the main body. As such, the detection module is allowed to displace by means of the extra preserved length of the slidable member, allowing the detection module to move back and forth in the opening of the main body to provide an effect of changing the site where detection is to be made for detecting physiological signals of different sites of the human body. Further, with such an operatively-disconnected arrangement, the stability of the detection module can be ensured and the phenomenon of inflaming caused by the detection module being retained on and depressing a fixed location of the skin, which may lead to discomfort of human body, can be eliminated. Thus, overall utilization can be improved.

A secondary object of the present invention is to provide a physiological detection structure, in which the main body is coupled to an internal layer of a fabric article so that the main body is fixedly combined to the fabric article. The combination is achieved with one of sewing, ultrasonic means, thermal fusion, and adhesive. The fabric article can be one of a garment, trousers, gloves, underwear, a vest, a corsage, and a tube-top. As such, when the fabric article is worn on a human body, the arrangement that the fabric article itself is formed through weaving or knitting an elastic material or an elastic body is coupled to each of two ends of the main body helps bond and attach the main body that is combined to the internal layer of the fabric article to the skin of the human body and allows the detection module that is exposed outside the opening of the main body to tightly attach to a site of the skin where detection is to be made, providing an effect of having the detection module showing a trend of moving toward the surface layer of the human body, improving the attachability of the contact face of the detection module to the surface layer of the human body, and enhancing the contact engagement with the surface layer of the human body. Thus, overall utilization can be improved.

A further object of the present invention is to provide a physiological detection structure, in which the slidable member comprises an extra preserved length that includes at least one elastic section, wherein, alternatively, an elastic section is coupled to each of two ends of the slidable member at two sides of the detection module and the detection module is mounted to a middle portion of the slidable member that has no elasticity. The elastic sections of the two ends of the slidable member that are arranged at two sides of the detection module allow the detection module to displace in order to keep the detection module of the slidable member that is exposed outside the openings of the main body flat for easy adjustment of the detection site of the detection module and providing the extra preserved length of the slidable member with an effect of flexible adjustment. Thus, overall utilization can be improved.

To achieve above objects, the present invention provides a physiological detection structure, which comprises: a main body, a slidable member, and a detection module. The main body comprises at least one opening formed therein. The slidable member is combined with a surface of the main body and comprises an extra preserved length. The detection module is combined with the slidable member and is exposed outside the opening of the main body in such a way that the detection module is displaceable through the extra preserved length of the slidable member.

### BRIEF DESCRIPTION OF THE DRAWINGS

The present invention can be fully understood from the following detailed description and preferred embodiments with reference to the accompanying drawings, in which:
Figure 1 is a perspective view shows a first embodiment of the present invention;
Figure 2 is an exploded view of the first embodiment of the present invention;
Figure 3 is a perspective view illustrating displacement of a detection module of the first embodiment of the present invention;
Figure 4 is a perspective view showing a film layer coupled to an inside surface of an opening of the first embodiment of the present invention;
Figure 5 is a perspective view shows a second embodiment of the present invention;
Figure 6 is a perspective view illustrating displacement of a detection module of the second embodiment of the present invention;
Figure 7 is a perspective view showing a third embodiment of the present invention;
Figure 8 is a perspective view illustrating displacement of a detection module of the third embodiment of the present invention;
Figure 9 is a schematic view showing a main body of a fourth embodiment of the present invention combined with a fabric article;
Figure 10 is a schematic view showing a film layer coupled to an inside surface of an opening of the main body of the fourth embodiment of the present invention that is combined with the fabric article;
Figure 11 is a perspective view illustrating displacement of a detection module of the main body of the fourth embodiment of the present invention that is combined with the fabric article;
Figure 12 is a schematic view illustrating elastic bodies coupled to two ends of the main body of the fourth embodiment of the present invention that is combined with the fabric article.

### DETAILED DESCRIPTION OF THE PREFERRED EMBODIMENTS

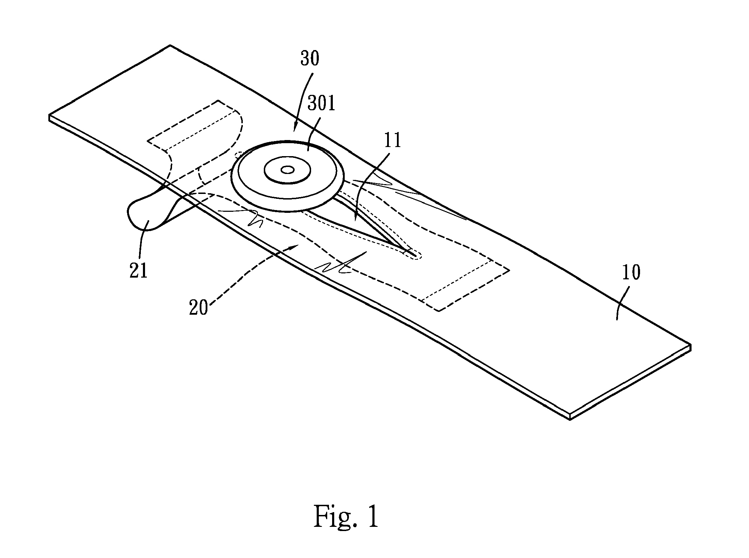

Referring to Figures 1-12, embodiments of the present invention are shown, in which a preferred form of a physiological detection structure according to the present invention is applicable to detection of variation of a physiological signal of a surface layer of a human body or that of an inclination angle of a human body and also provides an effect of changing the site where detection is made for detecting physiological signals of different sites of the human body and also provides an operatively-disconnected arrangement to improve stability of the detection module and eliminating the phenomenon of inflaming caused by long contacting and depressing a fixed location of the skin or discomfort of human body caused by being long contacted and depressed, thereby improving overall utilization.

A first embodiment of the physiological detection structure according to the present invention comprises a main body 10, a slidable member 20, and a detection module 30 (as shown in Figure 1). The main body 10 comprises at least one opening 11 formed therein. The opening 11 may extend transversely or longitudinally. (In the instant embodiment, a transverse opening is taken as an example.) The main body 10 can be one of a piece of fabric, a woven article, and a membrane. (In the instant embodiment, a piece of fabric is used as an example for the present invention.) The membrane can be a plastic membrane, a metal membrane, a carbon membrane, or a membrane made of other materials. Further, the slidable member 20 is combined to a surface of the main body 10 (wherein the slidable member 20 that is made of a non-elastic material is taken as an example in the instant embodiment) and the combination is achieved with one of sewing, ultrasonic means, thermal fusion, and adhesive, or alternatively, two ends of the slidable member 20 and the main body 10 are respectively provided with engageable fastening sections (such as buttons, snaps, hook and loop fasteners, and the likes). (In the instant embodiment, sewing is taken as an example.) The slidable member 20 is provided with an extra preserved length 21 to provide the slidable member 20 with an effect of being displaceable.

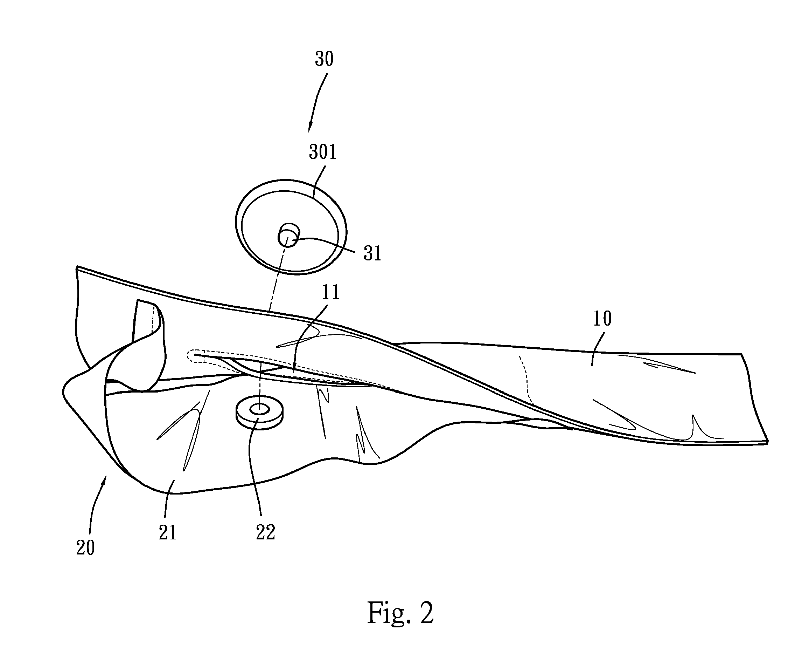

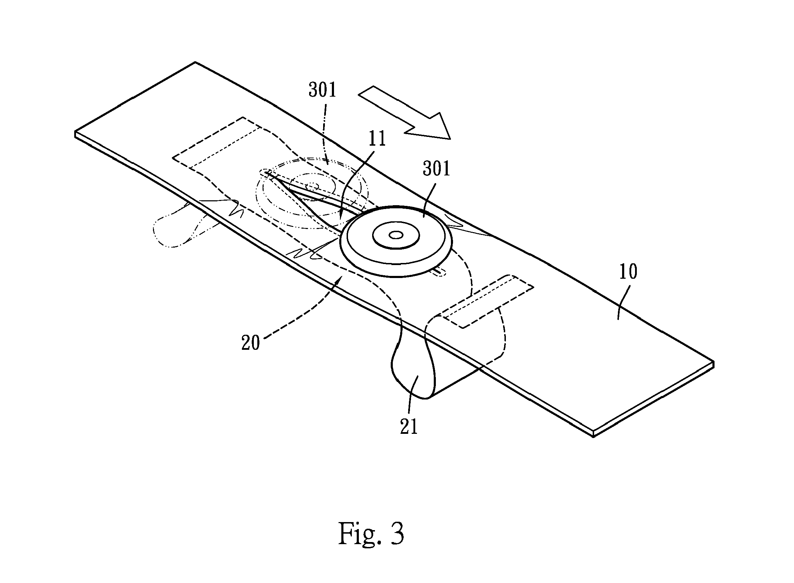

Further, the detection module 30 is combined to the slidable member 20 and the combination is achieved by providing the detection module 30 and the slidable member 20 respectively with mateable coupling sections 22, 31 (such as snaps, female-male pairs, and the likes), see Figure 2, or alternatively, the detection module 30 is directly mounted to the slidable member 20 (by means of for example one of sewing, ultrasonic means, thermal fusion, and adhesive). The detection module 30 is exposed outside the opening 11 of the main body 10. The detection module 30 comprises a contact face and the contact face of the detection module 30 is provided and arranged to contact a surface layer of a human body in order to detect a physiological signal of the surface layer of the human body. In this way, the detection module 30 is allowed to slide in the opening 11 of the main body 10 by means of the extra preserved length 21 of the slidable member 20 so as to provide an effect of changing the site where detection is made (as shown in Figure 3).

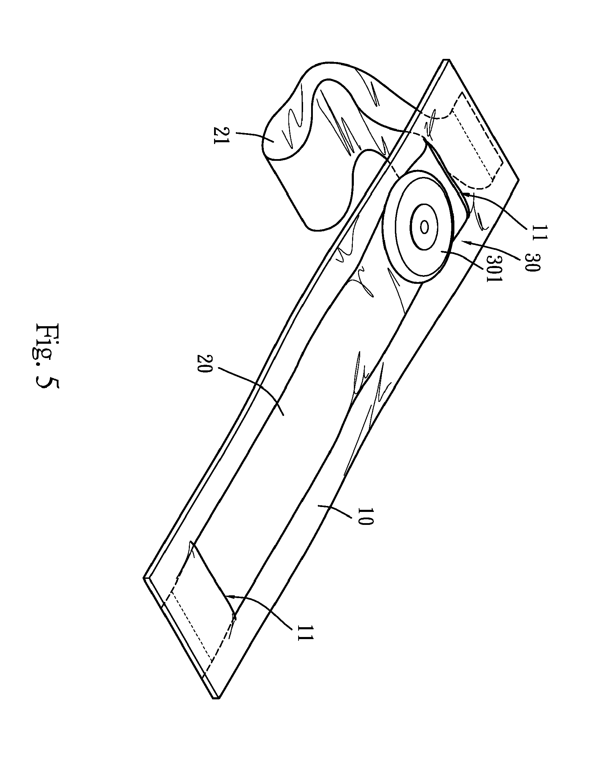

A second embodiment of the physiological detection structure according to the present invention comprises a main body 10, a slidable member 20, and a detection module 30 (as shown in Figure 5). The main body 10 comprises two opening 11 formed therein. The two openings 11 may extend transversely or longitudinally. (In the instant embodiment, two longitudinal openings 11 are illustrated, as an example). The main body 10 can be one of a piece of fabric, a woven article, and a membrane. (In the instant embodiment, a piece of fabric is used as an example for the present invention.) The membrane can be a plastic membrane, a metal membrane, a carbon membrane, or a membrane made of other materials. Further, the slidable member 20 is combined to a surface of the main body 10 (wherein the slidable member 20 that is made of a non-elastic material is taken as an example in the instant embodiment) and the combination is achieved with one of sewing, ultrasonic means, thermal fusion, and adhesive, or alternatively, two ends of the slidable member 20 and the main body 10 are respectively provided with engageable fastening sections (such as buttons, snaps, hook and loop fasteners, and the likes). (In the instant embodiment, sewing is taken as an example.) The slidable member 20 is provided with an extra preserved length 21 to provide the slidable member 20 with an effect of being displaceable.

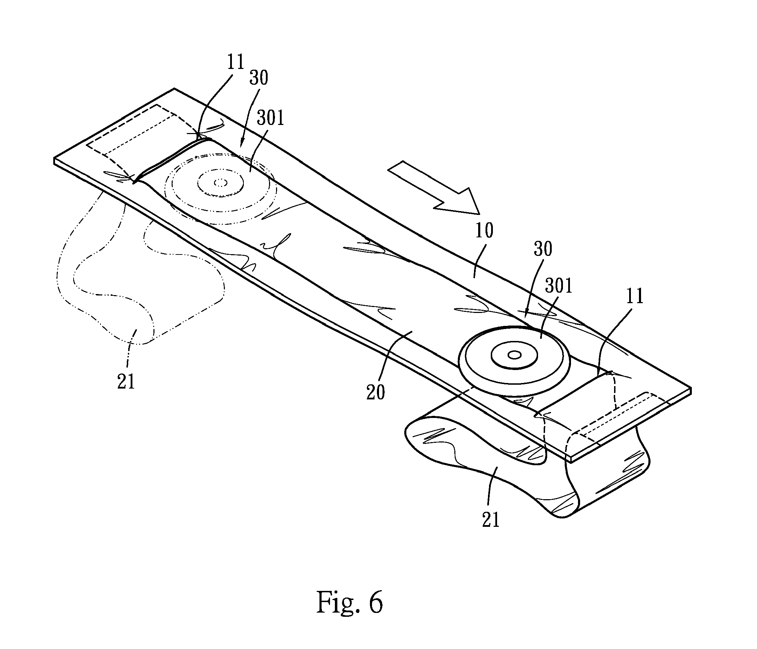

Further, the detection module 30 is combined to the slidable member 20 and the combination is achieved by providing the detection module 30 and the slidable member 20 respectively with mateable coupling sections 22, 31 (such as snaps, female-male pairs, and the likes), see Figure 2, or alternatively, the detection module 30 is directly mounted to the slidable member 20 (by means of for example one of sewing, ultrasonic means, thermal fusion, and adhesive). The detection module 30 is exposed outside the openings 11 of the main body 10. (In the instant embodiment, the slidable member 20 is set through the two longitudinal openings 11 of the main body 10 to allow the detection module 30 to be located outside and between the two openings 11 and thus exposed.) The detection module 30 comprises a contact face and the contact face of the detection module 30 is provided and arranged to contact a surface layer of a human body in order to detect a physiological signal of the surface layer of the human body. In this way, the detection module 30 is allowed to slide by means of the extra preserved length 21 of the slidable member 20 located inside the main body 10 so as to provide the detection module 30 with an effect of changing the site where detection is made between the two openings 11 of the main body 10 (as shown in Figure 6).

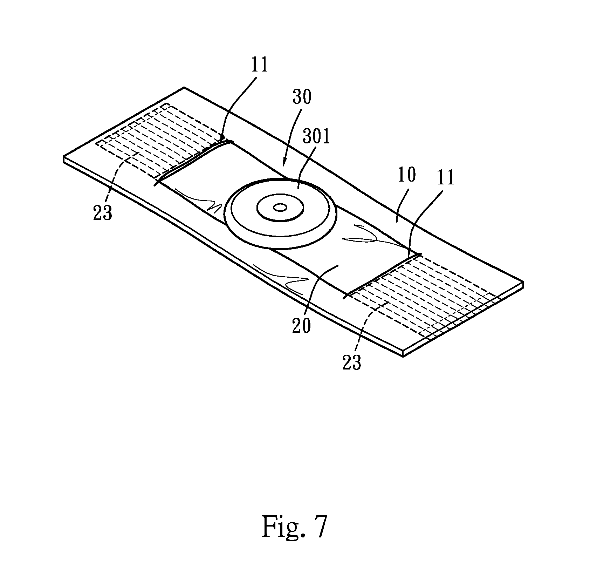

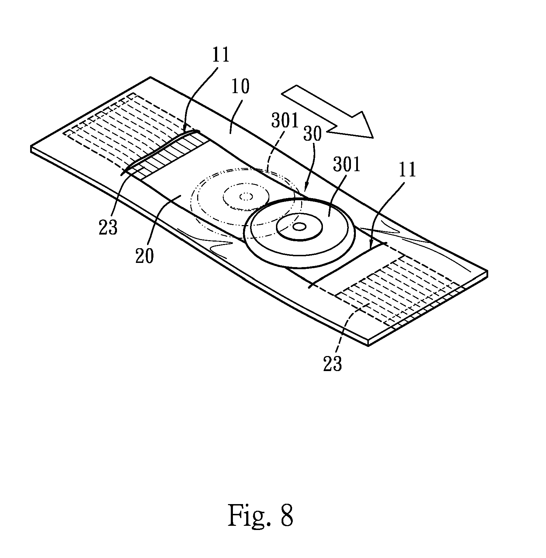

A third embodiment of the physiological detection structure according to the present invention comprises a main body 10, a slidable member 20, and a detection module 30 (as shown in Figure 7) and has a structural arrangement that is similar to those of the first and second embodiments with a primary difference being that the extra preserved length 21 of the slidable member 20 of the first and second embodiments is additionally provided with at least one elastic section 23. In the embodiment, an example that includes two openings 11 is presented. (However, the present invention is not limited to such an example, and is also applicable to an example including one opening 11.) Alternatively, the elastic section 23 can be provided at each of two ends of the slidable member 20 at two sides of the detection module 30 and the detection module 30 is mounted to a middle portion of the slidable member 20 that has no elasticity. The elastic section 23 may be combined with the slidable member 20 during the weaving or knitting thereof or, alternatively, any suitable means of combination may be adopted to combine the elastic section 23 and the slidable member 20 together. The combination may be one of sewing, ultrasonic means, thermal fusion, and adhesive. The elastic sections 23 of the two ends of the slidable member 20 that are arranged at two sides of the detection module 30 allow the detection module 30 to displace (as shown in Figure 8) in order to keep the detection module 30 of the slidable member 20 that is exposed outside the openings 11 of the main body 10 flat for easy adjustment of the detection site of the detection module 30 and providing the extra preserved length 21 of the slidable member with an effect of flexible adjustment.

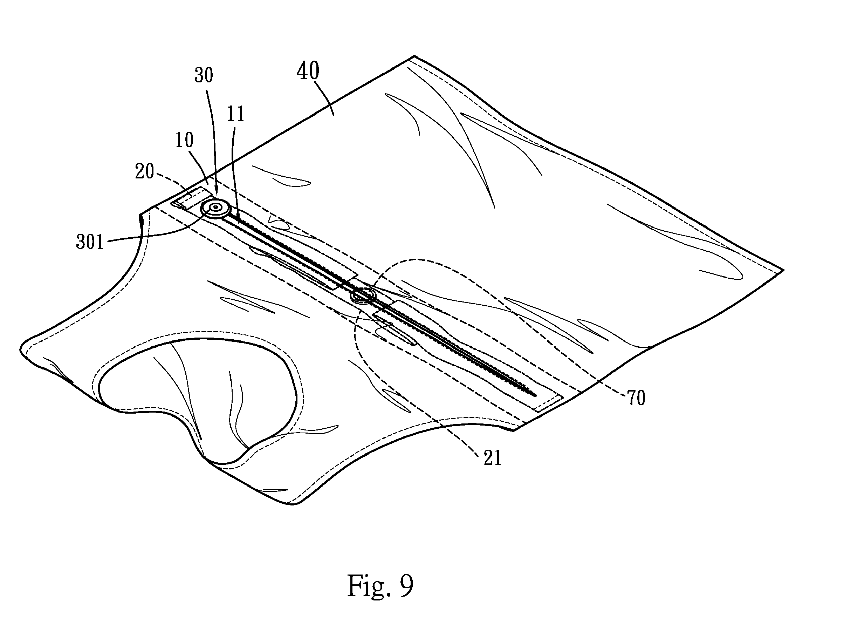

A fourth embodiment of the physiological detection structure according to the present invention comprises a main body 10, a slidable member 20, and a detection module 30 and has a structural arrangement that is similar to those of the first, second, and third embodiments with a primary difference being that a fabric article 40 is provided for combination with each of the first, second, and third embodiments, wherein the main body 10 is combined with an internal layer of the fabric article 40 (as shown in Figure 9) and the combination is achieved with one of sewing, ultrasonic means, thermal fusion, and adhesive. The fabric article 40 can be one of a garment, trousers, gloves, underwear, a vest, a corsage, and a tube-top and in the instant embodiment, a vest is taken as an example of the fabric article 40 for illustration, wherein the fabric article 40 in the form of a vest is wearable on a human body (the fabric article 40 in the form of a vest being shown inside out in the drawings) to establish contact engagement with the surface layer of the human body so as to allow the main body 10 that is combined to the vest of the fabric article 40 to get tight engagement with a specific site of the surface layer of the human body, such as chest or the pit of the stomach, with the detection module 30 that is exposed outside the opening 11 of the main body 10 contacting the surface layer of the human body. The fabric article 40 is formed through weaving or knitting an elastic material or a non-elastic material. When the fabric article 40 is formed by weaving or knitting an elastic material, the elastic material of the fabric article 40 helps bond the main body 10 that is combined to the internal layer of the fabric article 40 to the surface layer of the human body and allows the detection module 30 that is exposed outside the opening 11 of the main body 10 to tightly attach to a site of the surface layer of the human body where detection is to be made, providing an effect of having the detection module 30 showing a trend of moving toward the surface layer of the human body, improving the attachability of the contact face of the detection module 30 to the surface layer of the human body, and enhancing the contact engagement with the surface layer of the human body.

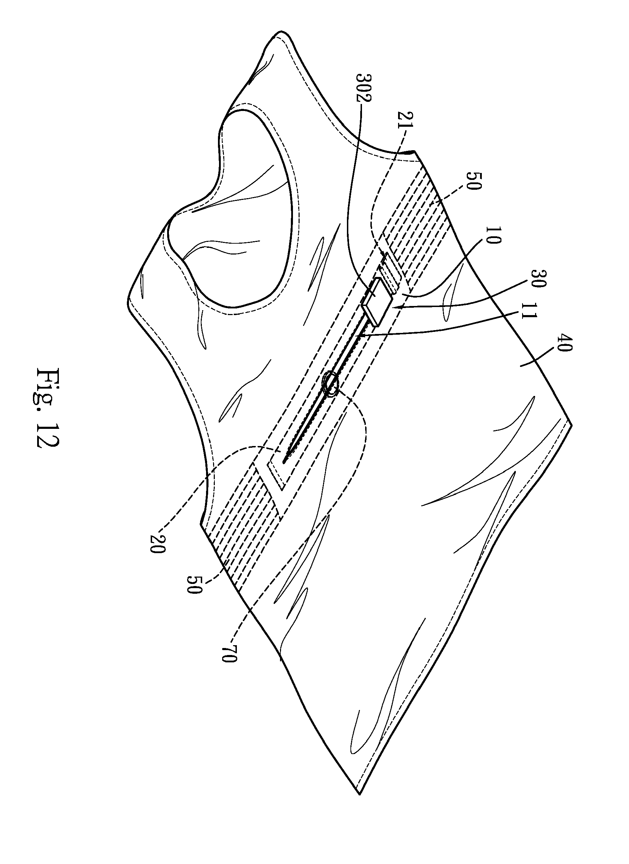

In the above-discussed fourth embodiment, when the fabric article 40 is formed through weaving or knitting a non-elastic material, an elastic body 50 may be additionally coupled to each of two ends of the main body 10 (as shown in Figure 12). The combination is achieved with one of sewing, ultrasonic means, thermal fusion, and adhesive. The elastic bodies 50 that are coupled to the two ends of the main body 10 may be extended to two sewing lines of the fabric article 40 to allow the elastic bodies 50 to retain the main body 10 on the fabric article 40 and allow the detection module 30 that is exposed outside the opening 11 of the main body 10 to tightly attach to a site of the surface layer of the human body where detection is to be made, providing an effect of having the detection module 30 showing a trend of moving toward the surface layer of the human body, improving the attachability of the contact face of the detection module 30 to the surface layer of the human body, and enhancing the contact engagement with the surface layer of the human body.

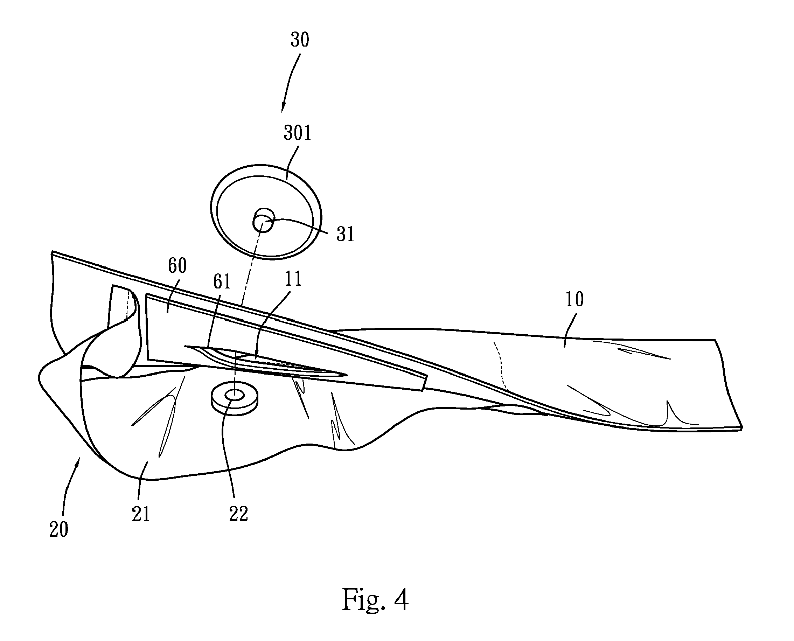

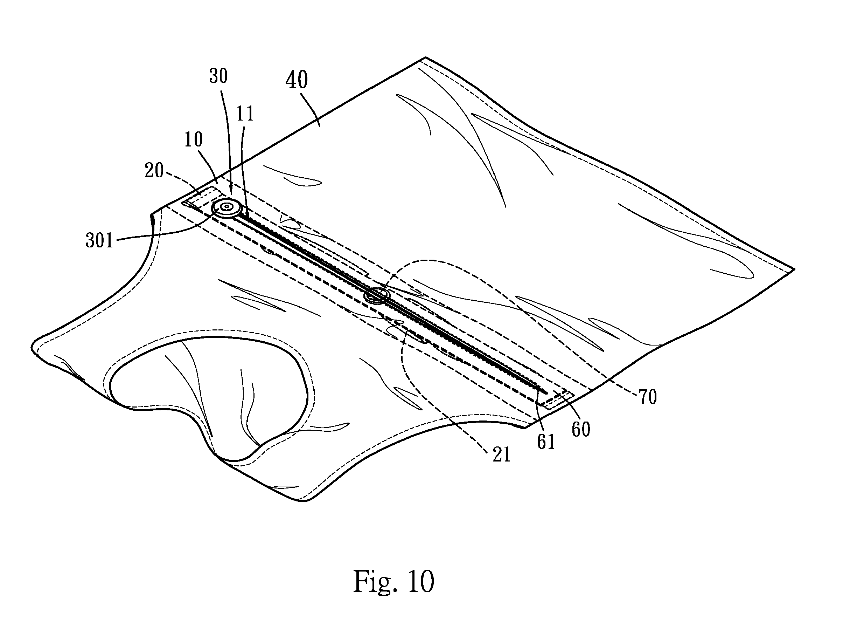

Further, in the above-described first, second, and third embodiments of the physiological detection structure according to the present invention, the opening 11 of the main body 10 (which in the instant embodiment is illustrated as a single transverse opening) may be constructed in such a way that the opening 11 of the main body 10 has an inside surface to which a film layer 60 (as shown in Figures 4 and 10) is coupled or combined. The combination can be achieved with one of sewing, ultrasonic means, thermal fusion, and adhesive and the film layer 60 is provided with an opening 61 corresponding to the opening 11 of the main body 10 so as to allow the detection module 30 that is mounted on the slidable member 20 to extend through the opening 61 of the film layer 60 and the opening 11 of the main body 10 for being exposed outside. The film layer 60 can be one of a plastic piece, a fabric piece (that may be additionally processed to provide stiffness, such as a fabric woven or knitted with acrylic yarns), and a membrane (which can be a plastic membrane, a metal membrane, a carbon membrane, or a membrane made of other materials) in order to improve the stiffness or rigidity of the opening 11 of the main body 10 to allow for easy displacement or sliding of the detection module 30 within the opening 11 of the main body 10 and to prevent the slidable member 20 from being easily pulled out of the opening 11 of the main body 10 so as to prevent the detection module 30 from being separated from a desired displacement trace and thus failing the operation thereof.

Further, in an embodiment of the opening 11 of the main body 10, two film layers (not shown) may be provided and combined with the inside surface of the opening 11 of the main body 10. The combination can be achieved with one of sewing, ultrasonic means, thermal fusion, and adhesive. The two film layers are respectively set at left and right sides of the opening 11 of the main body 10 to define a slit (not shown) in such a way that the opening 11 of the main body 10 corresponds to the slit, thereby allowing the detection module 30 that is mounted on the slidable member 20 to extend through the slit of the two film layers and the opening 11 of the main body 10 to get exposed outside. The two film layers can each be one of a plastic piece, a fabric piece (that may be additionally processed to provide stiffness, such as a fabric woven or knitted with acrylic yarns), and a membrane (which can be a plastic membrane, a metal membrane, a carbon membrane, or a membrane made of other materials) in order to improve the stiffness or rigidity of the opening 11 of the main body 10 to allow for easy displacement or sliding of the detection module 30 within the opening 11 of the main body 10 and to prevent the slidable member 20 from being easily pulled out of the opening 11 of the main body 10 so as to prevent the detection module 30 from being separated from a desired displacement trace and thus failing the operation thereof.

Further, in the above-described first, second, third, and fourth embodiments of the elastic physiological detection structure according to the present invention, the detection module 30 can be constructed to comprise, in a first example, an electrode plate 301 (as shown in Figures 1, 5, 7, and 9) for detecting a physiological signal of the surface layer of the human body. The electrode plate 301 is formed by weaving or knitting a plurality of non-conductive fiber yarns and a plurality of conductive fiber yarns; or alternatively, the electrode plate 301, in the entirety thereof, is formed solely of a plurality of conductive fiber yarns through weaving or knitting so as to make the entirety of the electrode plate 301 electrically conductive. Further, the plurality of conductive fiber yarns of the electrode plate 301 is woven or knitted to form a conductive zone. The conductive zone of the electrode plate 301 is positionable against and thus in tight contact engagement with the skin of the human body in order to detect a physiological signal. The physiological signal can be one of body temperature, heartbeat, pulse, and breath.

Further, in the above-described first, second, third, and fourth embodiments of the physiological detection structure according to the present invention, the detection module 30 can be constructed to comprise, in a second example, an inclination detection chip and a microcontroller 302 (as shown in Figure 12). The microcontroller 302 is connected to the inclination detection chip so that the microcontroller 302 may detect a variation of the inclination detection chip. The inclination detection chip can be a three-axis acceleration transducer (such as a three-axis low-g micro-machined accelerometer). The three-axis acceleration transducer is operable to detect/calculate inclination angles and accelerations in three axes of X, Y, and Z and the microcontroller 302 is operable to periodically detect and transmit these values. The inclination angle can be used to detect an event of dizziness or fall by setting a change exceeding a threshold of +/-1.0 degree, +/-1.5 degrees, or +/-2.0 degrees. The microcontroller 302 can be set to conduct detection at a fixed time interval with a fixed number of detection operations. For example, abnormal inclination may be identified by means of an average of successively detected values within a period of 30 seconds being determined exceeding +/-1.0 degree (or +/-1.5 degrees or +/-2.0 degrees). Or alternatively, a nine-axis body position transducer (not shown) may be used. The nine-axis body position transducer comprises a three-axis acceleration sensor, three-axis magnetic field sensor, and a three-axis gyro sensor with the range of magnetic field being ±1.3/1.9/2.5/4.0/4.7/5.6/8.1 gausses, the range of the acceleration being ±2g/±4g/±8g, and the range of the gyro being ±250/500/2000 dps. Abnormality can be identified by determining by monitoring and detecting a value of a body position according to the above-mentioned ranges. When the detected value exceeds the ranges, abnormal inclination is identified. When a variation of the inclination or body position exceeds a setting value or a threshold, the microcontroller 302 issues, through a wireless signal transmitter (not shown), a signal.

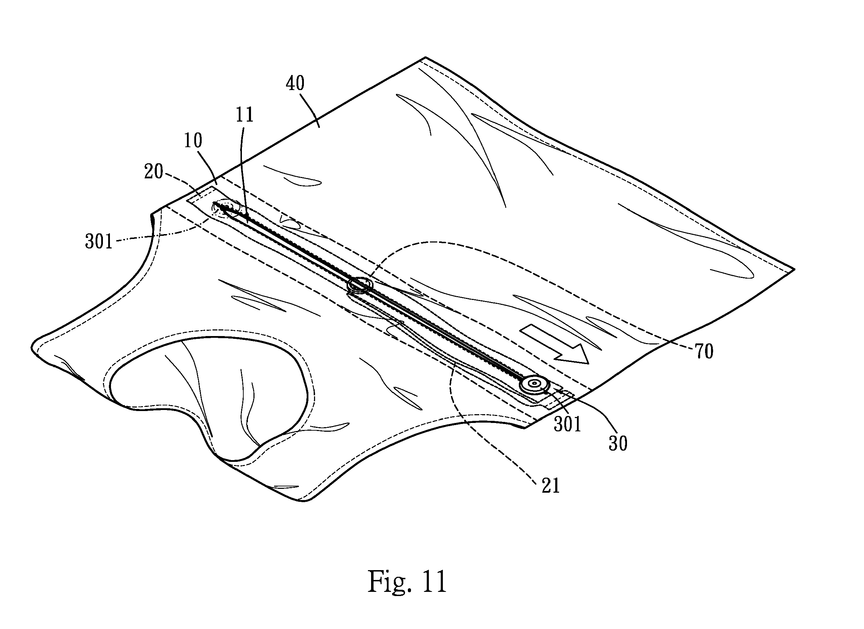

Further, in the above-described fourth embodiment of the physiological detection structure according to the present invention, the fabric article 40 is provided with a signal transmission terminal 70 (as shown in Figure 11) and the signal transmission terminal 70 is coupled to at least one detection module 30 so that the variation of the physiological signal or the inclination angle of the human body detected by the detection module 30 can be transmitted to the signal transmission terminal 70. In addition, the signal transmission terminal 70 may be coupled to a signal transmitter (not shown) and the signal transmitter comprises a wireless transmission module that transmits, in a wireless manner, the variation of the physiological signal or the inclination angle of the human body to an electronic device (such as a smart mobile phone, a tablet computer, a notebook computer, a desktop computer, and medical facility) or to the cloud for realization of the detection result and to provide preventive medical treatment.

Further, in the above-described first, second, third, and fourth embodiments of the physiological detection structure according to the present invention, a combined assembly of a main body 10, a slidable member 20, and a detection module 30 is provided in such a way that the main body 10 is provided with at least one opening 11 formed therein; the slidable member 20 is combined to a surface of the main body 10 and the slidable member 20 is provided with an extra preserved length 21; and the detection module 30 is combined to the slidable member 20 and the detection module 30 is exposed outside the opening 11 of the main body 10, whereby the detection module 30 is allowed to displace by means of the extra preserved length 21 of the slidable member 20, allowing the detection module 30 to move back and forth in the opening 11 of the main body 10 to provide an effect of changing the site where detection is to be made for detecting physiological signals of different sites of the human body. Further, with such an operatively-disconnected arrangement, the stability of the detection module 30 can be ensured and the phenomenon of inflaming caused by the detection module 30 being retained on and depressing a fixed location of the skin, which may lead to discomfort of human body, can be eliminated.

Further, the main body 10 may be coupled to an internal layer of a fabric article 40 so that the main body 10 is fixedly combined to the fabric article 40. The fabric article 40 can be one of a garment, trousers, gloves, underwear, a vest, a corsage, and a tube-top. As such, when the fabric article 40 is worn on a human body, the arrangement that the fabric article 40 itself is formed through weaving or knitting an elastic material or an elastic body 50 is coupled to each of two ends of the main body 10 helps bond and attach the main body 10 that is combined to the internal layer of the fabric article 40 to the skin of the human body and allows the detection module 30 that is exposed outside the opening 11 of the main body 10 to tightly attach to a site of the skin where detection is to be made, providing an effect of having the detection module 30 showing a trend of moving toward the surface layer of the human body, improving the attachability of the contact face of the detection module 30 to the surface layer of the human body, and enhancing the contact engagement with the surface layer of the human body.

Based on the above detailed description, those skilled in the art may appreciate that the present invention can achieve the above-discussed objectives. However, it is noted that the above description is made only to a preferred embodiment of the present invention and is not intending to limit the true scope where the present invention may be put into practice. Thus, simple and equivalent variations and modifications made on the disclosure of the specification and the attached claims are all considered within the scope of the present invention.

## Claims

1. A physiological detection structure, comprising:
a main body, which comprises at least one opening formed therein;
a slidable member, which is combined with a surface of the main body and comprises an extra preserved length; and
a detection module, which is combined with the slidable member and is exposed outside the opening of the main body in such a way that the detection module is displaceable through the extra preserved length of the slidable member.

2. The physiological detection structure as claimed in Claim 1, wherein the opening of the main body comprises a longitudinal opening or a transverse opening and the main body comprises one of a piece of fabric, a woven article, and a membrane.

3. The physiological detection structure as claimed in Claim 1 or 2, wherein the opening of the main body has an inside surface to which a film layer is combined, the film layer comprising an opening formed therein to correspond to the opening of the main body, the film layer comprising one of a plastic piece, a fabric piece, and a membrane.

4. The physiological detection structure as claimed in Claim 1 or 2, wherein the opening of the main body has an inside surface to which two film layers are combined in such a way that the two film layers are respectively located at left and right sides of the opening of the main body to define a slit, the opening of the main body corresponding to the slit, the film layers each comprising one of a plastic piece, a fabric piece, and a membrane.

5. The physiological detection structure as claimed in Claim 1, wherein extra preserved length of the slidable member comprises an elastic section.

6. The physiological detection structure as claimed in Claim 1, wherein the main body is combined to an internal layer of a fabric article and the fabric article comprises one of a garment, trousers, gloves, underwear, a vest, a corsage, and a tube-top and the fabric article is formed through weaving or knitting an elastic material or a non-elastic material

7. The physiological detection structure as claimed in Claim 1, wherein the main body has two ends to each of which an elastic body is coupled.

8. The physiological detection structure as claimed in Claim 1, wherein the combination of the slidable member and the main body is such that two ends of the slidable member and the main body are respectively provided with engageable fastening sections.

9. The physiological detection structure as claimed in Claim 1, 6, or 7, wherein the combination comprises one of sewing, ultrasonic means, thermal fusion, and adhesive.

10. The physiological detection structure as claimed in Claim 1, wherein the combination of the detection module and the slidable member is such that mateable coupling sections are respectively provided on the detection module and the slidable member.

11. The physiological detection structure as claimed in Claim 1, wherein the combination of the detection module and the slidable member is such that the detection module is directly mounted to the slidable member.

12. The physiological detection structure as claimed in Claim 1, wherein the detection module comprises an electrode plate that is adapted to detect a physiological signal of a surface layer of a human body and the electrode plate is connected to a signal transmission terminal.

13. The physiological detection structure as claimed in Claim 1, wherein the detection module comprises an inclination detection chip and a microcontroller, the microcontroller being connected to the inclination detection chip so that the microcontroller detects a variation of the inclination detection chip.
